# EUROPEAN PATENT APPLICATION

(11) **EP 0 568 254 A2**
(43) Date of publication of application: **03.11.1993**
(21) Application number: 93303075.1
(22) Date of filing: 21.04.1993
(51) Int. Cl.: C07D 233/06, C07D 233/16

(54) **Method for preparing 1,2-substituted imidazoline compositions**

(30) Priority: 29.04.1992 US 875464
(71) Applicant: Huntsman Corporation, Salt Lake City, Utah 84111-1053 (US)
(72) Inventor: Hollingsworth, Donald Ray, Austin, Texas 78723 (US); Edwards, Jeffrey Howard, Spring, Texas 77379 (US)
(74) Representative: Brock, Peter William

(57) **Abstract**

A 1,2-substituted imidazoline composition is prepared by reacting a polyamine mixture comprising triethylenetetramine or tetraethylenepentamine oligomers with an acidic compound selected from aliphatic monocarboxylic and dicarboxylic acids, and esters and mixtures thereof, at imidazoline reaction temperature and pressure. The polyamine mixture comprises 75 wt% or more of linear triethylenetetramine oligomer or 65 wt% or more of linear tetraethylenepentamine oligomer, and leads to improved yields of the 1,2-substituted imidazoline.

## Description

This invention relates to a method for preparing 1,2-substituted imidazoline compositions.

Compounds having an imidazoline ring structure substituted at positions -1 and -2 are well-known in the art. Imidazolines substituted at the 2-position with a long chain aliphatic group are cationic compounds, exhibiting surface active properties. They have been used as cationic emulsifiers, flotation agents, corrosion inhibitors and the like.

US-A-4709045 discloses a process for producing 1,2-substituted imidazoline compounds by the reaction of dialkylenetriamine with a higher fatty acid.

US-A-2878234 discloses diimidazoline compounds. These are formed by the reaction of a polyamine, such as triethylenetriamine or tetraethylenepentamine, with as aliphatic dibasic acid.

US-A-4855440 discloses stabilized imidazoline derivatives. The patent teaches that the numbering system for the imidazoline ring is conventional.

This invention relates to a method for making a 1,2-substituted imidazoline composition by reacting a polyamine mixture comprising triethylenetetramine or tetraethylenepentamine oligomers with an acidic compound selected from aliphatic monocarboxylic and dicarboxylic acids, and esters and mixtures thereof. According to the invention, the polyamine mixture comprises 75 wt% or more of linear triethylenetetramine oligomer or 65 wt% or more of linear tetraethylenepentamine oligomer.

As a result compositions, concentrated in 1,2-substituted imidazoline, are recovered from polyamine feedstocks. These compositions are suitable for curing epoxy resins without additional separation or concentration.

The polyamine mixture used according to the invention comprises linear triethylenetetramine or linear tetraethylenepentamine. These compounds are synthesized by the reaction of ethylenediamine and monoethanolamine in mole ratios of 1 to 5 moles of ethylenediamine per mole of monoethanolamine. The catalyst used is generally a thermally-activated, calcined pelleted titania having active phosphorous sites chemically bonded to the surface. The reaction is generally carried out as a continuous process of 250 to 400°C, and a pressure of 3.5 to 21 MPa (500 to 3000 psig) sufficient to maintain the reaction mixture in the liquid phase.

The preparation of these compounds is well documented in the patent literature. For example, US-A-4324917 teaches that a commercially available cation exchange resin was used to convert a 2/1 mole ratio mixture of ethylenediamine and monoethanolamine into linear products, at a temperature of 305°C and a pressure of 10.4 MPa (1500 psig), and a liquid hourly space velocity of 1 g/h/cm³ catalyst volume. The noncyclics content of the triethylenetetramine compounds was 96.8%.

For the purpose of clarity in interpretation, we define the terms triethylenetetramine and linear triethylenetetramine as follows. The catalytic reaction of ethylenediamine with monoethanolamine yields a product mixture comprising unreacted ethylenediamine and monoethanolamine, along with a number of linear, branched and cyclic reaction products. The product mixture is fractionally distilled to recover an unreacted fraction, which is recycled. Another fraction contains triethylenetriamine.

Triethylenetetramine is a generic term covering four compounds:
liner triethylenetetramine,
nitrilotrisethylamine,
diaminoethylpiperazine, and
piperazinoethylethylenediamine.

By definition, triethylenetetramine refers to the mixture of four compounds. Linear triethylenetetramine refers only to the linear product having the structural formula:

H₂NCH₂CH₂NHCH₂CH₂NHCH₂CH₂NH₂

Nitrilotrisethylamine has a branched structure: 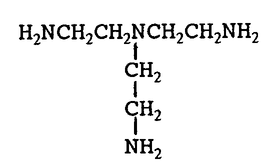
Diaminoethylpiperazine has the cyclic structure: 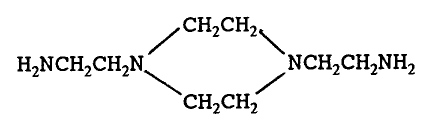
Piperazinoethylethylenediamine has the cyclic structure: 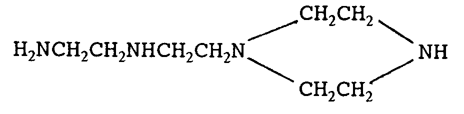
Correspondingly, tetraethylenepentamine is a generic term covering four compounds:
liner tetraethylenepentamine,
aminoethyltriethylenetriamine,
aminoethylpiperazinylethylethylenediamine, and
piperazinylethyldiethylenetriamine.

By definition, tetraethylenepentamine refers to the mixture of four compounds. Linear tetraethylenepentamine refers only to the linear product having the structure:

H₂NCH₂CH₂NHCH₂CH₂NHCH₂CH₂NHCH₂CH₂NH₂

Aminoethyltriethylenetetramine has a branched structure: 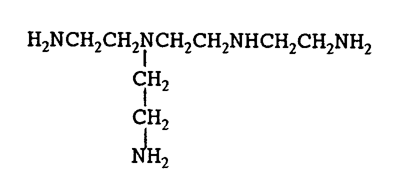
Aminoethylpiperazinylethylethylenediamine has the cyclic structure: 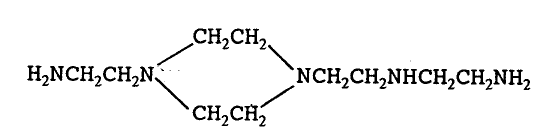
Piperazinylethyldiethylenetriamine has the cyclic structure: 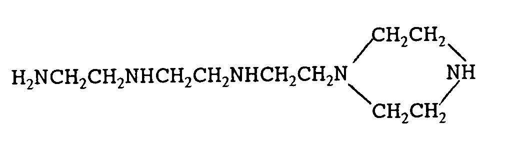
The linear oligomers have the greatest commercial value. For that reason, the patent art describes a number of catalyst which are useful in improving the yield of the linear oligomers. These include US-A-4588842, US-A-4578518, and US-A-4578519. In general, these patents teach that, by reaction and fractional distillation, a triethylenetriamine mixture is produced which contains at least 75 wt.% of the linear triethylenetetramine. Process optimization yields a product which contains at least 80 wt% of the linear product. US-A-4324917 teaches that a product containing 96.8% of the noncyclic material has been made. Likewise, a tetraethylenepentamine mixture is produced containing at least 65 wt% of the linear oligomer. Process optimization yields a product containing 70 wt.% liner oligomer. These high concentration linear oligomer mixtures are the feedstocks used according to the invention for synthesizing 1,2-substituted imidazolines.

The other reactant employed in the method according to the invention is an acidic compound, selected from aliphatic monocarboxylic and dicarboxylic acids, corresponding esters, and mixtures thereof.

A preferred group of such acidic compounds is provided by fatty acids, herein defined as monobasic aliphatic, saturated, unsaturated or polyunsaturated acids containing from 8 to 30 carbon atoms, and often from 12 to 22 carbon atoms. Fatty acids are derived from vegetable fats or oils, though they can be recovered from fish or animal fats or oils, or can be entirely synthetic. Fatty acids derived from natural sources are usually a mixture of acids. For example tall oil fatty acid is a mixture consisting primarily of oleic acid and linoleic acid. Tall oil fatty acid is a by-product of digesting pinewood in the kraft paper process.

Representative fatty acids include: heptanoic, caprylic, caproic, decenoic, undecanoic, lauric, dodecanoic, tridecanoic, myristic, tetradecenoic, pentadecenoic, hexadecanoic, palmitic, heptadecenoic, stearic, oleic, nonadecanoic, eicosanoic, behenic and tetracosanoic acids. Fatty acid mixtures include fatty acids derived from tallow, soybean and coconut oil.

Esters, particularly alkyl esters, of these fatty acids, referred to as fatty esters, can also be used in the method according to the invention. These esters, include the esterification products with organic alcohols such as methanol, ethanol and propanol. The methyl ester is preferred.

Unsaturated fatty acids can be condensed to form dimer fatty acids. These are dicarboxylic acids. Other dicarboxylic acids that can be used include adipic, sebacic, azelaic, glutaric, succinic, oxalic, malonic and maleic acids. These dicarboxylic acids contain two carboxylic acid groups, both of which can react with polyamine, whereby to form diimidazolines. These diimidazolines are disclosed, for example, in US-A-2878234. These esters of these dimer fatty acids are also useful in the method according to the invention. Again the methyl ester and diester are preferred.

The imidazole compositions can be formed in a two step reaction. The relative amounts of reactants are such that the carboxylic acid or ester groups are present in excess over the stoichiometric amount required for reaction with the polyamine. Stoichiometry would dictate a carboxylic acid or ester:polyamine ratio of 1:1. In practice a ratio of 1.5:1 to 1.8:1 is used, so that all of the polyamine is reacted.

The initial condensation of the acid and polyamine may be carried out at a temperature of 120 to 200°C, under an inert atmosphere. This is followed by the cyclization reaction, carried out at a temperature of 195 to 290°C and at reduced pressure of 0.1 to 6.7 kPa (1 to 50 mm Hg), preferably 1.3 to 4.0 kPa (10 to 30 mm Hg). The extent of cyclization can be followed by analyzing the imidazoline to amide ratio by infrared spectroscopy (IR). Heating is terminated when the imidazoline to amide ratio produces an optimum yield. Alternatively, heating may be terminated when sufficient time has passed for optimum yield to be achieved, based on past experience. The maximum time required is generally 10 hours, and typically 3 to 8 hours. The liquid reaction product is allowed to cool to room temperature, and sampled for quality control.

It has been found that polyamine mixtures comprising these high concentrations of linear polyamine provide reaction products that do not require separation or other purification before use as epoxy resin curing agents.

This invention is shown by way of Example.

### EXAMPLE 1

To a 4-neck flask equipped with a mechanical stirrer, Dean-Stark trap and condenser, thermometer and argon inlet was added 125g (0.66 mol) of tetraethylenepentamine (TEPA) comprising 73.8% of linear TEPA (TEXLIN® 400). To this was added 375g of tall oil fatty acid (TOFA) (FA2 Acintol, 1.32 mol), and the mixture was heated at 190°C for 3 hours. The material was then cooled and analyzed with the following results:

| | |
|---|---|
| % Imidazoline, by NMR | 15.6 |
| Total Amine, meq/g | 3.9 |
| Primary Amine, meq/g | 0.8 |
| Secondary Amine, meq/g | 2.6 |
| Tert. Amine, meq/g | 0.6 |
| Water, Wt% | 0.4 |
| Color, Gardner | 7 |
| Viscosity @ 25°C, cps | 1481 |

### EXAMPLE 2 - COMPARATIVE

Example 1 was repeated with TEPA containing 51.5% of linear TEPA. This imidazoline product was analyzed with the following results:

| | |
|---|---|
| % Imidazoline, by NMR | 8.3 |
| Total Amine, meq/g | 3.8 |
| Primary Amine, meq/g | 1.8 |
| Secondary Amine, meq/g | 1.1 |
| Tert. Amine, meq/g | 0.9 |
| Water, Wt% | 0.5 |
| Color, Gardner | 8 |
| Viscosity @ 25°C, cps | 1672 |

### EXAMPLE 3 - COMPARATIVE

Example 1 was repeated with TEPA containing 48.5% of linear TEPA. This imidazoline product was analyzed with the following results:

| | |
|---|---|
| % Imidazoline, by NMR | 7.2 |
| Total Amine, meq/g | 3.8 |
| Primary Amine, meq/g | 0.5 |
| Secondary Amine, meq/g | 2.3 |
| Tert. Amine, meq/g | 1.0 |
| Water, Wt% | 0.3 |
| Color, Gardner | 7 |
| Viscosity @ 25°C, cps | 1770 |

### EXAMPLE 4

Example 1 was repeated at a reaction temperature of 210°C. This imidazoline product was analyzed with the following results:

| | |
|---|---|
| % Imidazoline, by NMR | 25.7 |
| Total Amine, meq/g | 4.0 |
| Primary Amine, meq/g | 0.5 |
| Secondary Amine, meq/g | 2.3 |
| Tert. Amine, meq/g | 1.0 |
| Water, Wt% | 0.3 |
| Color, Gardner | 7 |
| Viscosity @ 25°C, cps | 1770 |

### EXAMPLE 5 - COMPARATIVE

Example 4 was repeated with TEPA containing 51.5% linear TEPA. This imidazoline product was analyzed with the following results:

| | |
|---|---|
| % Imidazoline, by NMR | 20.6 |
| Total Amine, meq/g | 3.8 |
| Primary Amine, meq/g | 0.6 |
| Secondary Amine, meq/g | 2.2 |
| Tert. Amine, meq/g | 1.1 |
| Water, Wt% | 0.23 |
| Color, Gardner | 6 |
| Viscosity @ 25°C, cps | 2096 |

### EXAMPLE 6 - COMPARATIVE

Example 4 was repeated with TEPA containing 48.5% linear TEPA. This imidazoline product was analyzed with the following results:

| | |
|---|---|
| % Imidazoline, by NMR | 18.9 |
| Total Amine, meq/g | 3.9 |
| Primary Amine, meq/g | 0.6 |
| Secondary Amine, meq/g | 2.1 |
| Tert. Amine, meq/g | 1.1 |
| Water, Wt% | 0.24 |
| Color, Gardner | 6 |
| Viscosity @ 25°C, cps | 1277 |

### EXAMPLE 7

To a 4-neck flask equipped with a mechanical stirrer, condenser, thermometer and argon inlet was added 101.6g (0.69 mol) of triethylenetetramine (TETA) containing 84.1% of linear TETA (TEXLIN® 300). To this was added 161.5g of C₁₆-C₆₀ dimer acid having an average molecular weight of C₃₈ (Unidyme 22, 0.28 mol). The mixture was heated at 165°C for 2 hours. A vacuum of 10 kPa (75mm of Hg) was applied and the temperature was increased to 235°C for 5 hours. The material was then cooled and analyzed with the following results:

| | |
|---|---|
| Imidazoline/Amide Ratio, by IR | 8.2 |
| Total Amine, meq/g | 8.5 |
| Tert. Amine, meq/g | 0.9 |
| Color, Gardner | 7 |
| Viscosity @ 25°C, cps | 1202 |

### EXAMPLE 8 - COMPARATIVE

Example 7 was repeated with TETA containing 64.5% of linear TETA. This imidazoline product was analyzed with the following results:

| | |
|---|---|
| Imidazoline/Amide Ratio, by IR | 5.9 |
| Total Amine, meq/g | 6.6 |
| Tert. Amine, meq/g | 1.6 |
| Color, Gardner | 10 |
| Viscosity @ 25°C, cps | 3198 |

### EXAMPLE 9 - COMPARATIVE

Example 7 was repeated with TETA containing 71.3% of linear TETA. This imidazoline product was analyzed with the following results:

| | |
|---|---|
| Imidazoline/Amide Ratio, by IR | 5.1 |
| Total Amine, meq/g | 7.8 |
| Tert. Amine, meq/g | 1.1 |
| Color, Gardner | 9 |
| Water, wt% | 0.14 |
| Viscosity @ 25°C, cps | 2083 |

### EXAMPLE 10 - COMPARATIVE

Example 7 was repeated with TETA containing 63.0% of linear TETA. This imidazoline product was analyzed with the following results:

| | |
|---|---|
| Imidazoline/Amide Ratio, by IR | 3.0 |
| Total Amine, meq/g | 7.6 |
| Tert. Amine, meq/g | 1.3 |
| Color, Gardner | 7 |
| Water, wt% | 0.18 |

## Claims

1. A method for making a 1,2-substituted imidazoline composition by reacting a polyamine mixture comprising triethylenetetramine or tetraethylenepentamine oligomers with an acidic compound selected from aliphatic monocarboxylic and dicarboxylic acids, and esters and mixtures thereof, at imidazoline reaction temperature and pressure, characterised in that said polyamine mixture comprises 75 wt% or more of linear triethylenetetramine oligomer or 65 wt% or more of linear tetraethylenepentamine oligomer.

2. A method according to Claim 1, characterised in that the polyamine mixture comprises 80 wt% or more of linear triethylenetetramine oligomer.

3. A method according to Claim 1, characterised in that the polyamine mixture comprises 70 wt% or more of linear tetraethylenepentamine oligomer.

4. A method according to any one of Claims 1 to 3, characterised in that the reaction conditions comprise a first step carried out at a temperature of 120 to 200°C under an inert atmosphere, and a second step carried out at a temperature of 195 to 290°C and a pressure of 0.1 to 6.7 KPa (1 to 50 mm Hg).

5. A method according to any one of Claims 1 to 4, characterised in that the acidic compound is a C₈ to C₃₀ fatty acid, an ester thereof, a dimer fatty acid thereof, a fatty ester thereof or a mixture thereof.
